# Europäisches Patentamt
## European Patent Office
## Office européen des brevets

(19)

(11) Veröffentlichungsnummer: **0 093 378**
**B1**

(12) **EUROPÄISCHE PATENTSCHRIFT**

(45) Veröffentlichungstag der Patentschrift:
**12.08.87**

(51) Int. Cl.⁴: **A 61 F 2/36**

(21) Anmeldenummer: **83104071.2**

(22) Anmeldetag: **26.04.83**

(54) **Femorale Hüftgelenkprothese.**

(30) Priorität: **03.05.82 DE 3216539**

(43) Veröffentlichungstag der Anmeldung:
**09.11.83 Patentblatt 83/45**

(45) Bekanntmachung des Hinweises auf die Patenterteilung:
**12.08.87 Patentblatt 87/33**

(84) Benannte Vertragsstaaten:
**CH DE FR GB IT LI SE**

(56) Entgegenhaltungen:
**DE-A-2 839 092**
**DE-A-2 933 229**
**DE-C-837 294**
**DE-U-8 124 912**
**FR-A-1 278 359**
**GB-A-2 005 546**
**US-A-2 719 522**
**US-A-3 067 740**
**US-A-3 704 769**

(73) Patentinhaber: **Waldemar Link GmbH & Co,**
**Barkhausenweg 10, D-2000 Hamburg 63 (DE)**

(72) Erfinder: **Keller, Arnold, An der Naherfurth 5,**
**D-2061 Kayhude (DE)**

(74) Vertreter: **Glawe, Delfs, Moll & Partner**
**Patentanwälte, Postfach 26 01 62 Liebherrstrasse**
**20, D-8000 München 26 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine femorale Hüftgelenkprothese mit einem zur zementfreien Verankerung im Femur geeigneten Schaft, der eine Mehrzahl von von einem schlanken Schaftkern vorspringenden Längsrippen aufweist, von denen die dorsal und ventral vorgesehenen Rippen der medialen Seite eine Breitfläche zukehren und eine Vielzahl von Öffnungen enthalten.

Im Bereich des Femurknochens distal von den Trochantern ist die harte Außenschicht des Knochens, die Corticalis, verhältnismäßig dick und der Markkanal dünn. In diesem Bereich kann ein Prothesenschaft verhältnismäßig sicher verankert werden, weil eine direkte oder über Knochenzement vermittelte Kraftübertragung von der Prothese auf die Corticalis möglich ist. Im proximalen Femurbereich ist die sich trichterförmig erweiternde Corticalis sehr dünn und wird der Knochenquerschnitt im wesentlichen von einem feinlamellaren Trajektoriensystem des spongiösen Knochengewebes gebildet, das bei der Kraftübertragung von der Prothese her nur wesentlich geringeren spezifischen Belastungen ausgesetzt werden darf. Dieser Bereich, der für die Zwecke der vorliegenden Beschreibung als der trochantere Femurbereich bezeichnet wird, liegt etwa oberhalb der unteren Begrenzung des kleineren Trochanters. Wird die Prothese in diesem Bereich unter Vermittlung von Knochenzement verankert, so kann durch die Ausbildung der vor dem Einsetzen der Prothese ausgeraspelten Knochenhöhlung und der daraus resultierenden Form des Zementkörpers die Form und Größe der kraftübertragenden Fläche hinreichend bemessen werden. Wenn hingegen die Verankerung ohne Knochenzement erfolgt, muß der Prothesenschaft als solcher eine hinreichend große Kraftübertragungsfläche zur Verfügung stellen und daher in seinem proximalen Bereich stark verdickt sein, was in diesem Bereich zu großer Steifigkeit des Schafts führt. Angestrebt wird jedoch eine Annäherung der Schaftelastizität an diejenige des Knochens (Z.Orthop. 1979, S. 481), da nur so eine gleichmäßige Kraftübertragung möglich ist. Andernfalls kann es zu Lockerungserscheinungen wegen örtlicher Knochenrückbildung aufgrund von Über- bzw. Entlastung des Knochengewebes kommen.

Es ist ein Prothesenschaft der eingangs genannten Art bekannt (DE-A- 28 39 092), der als ein im Querschnitt kreuzförmiger, mit vier Längsrippen versehener, in den Knochenkanal eintreibbarer Nagel ausgebildet ist, der über den größten Teil seiner Länge konstante Querschnittsgestalt hat und lediglich am proximalen Ende eine zur Anpassung an die dort vorgesehene Krümmung und an dem Übergang zum Prothesenkragen eine etwas geänderte Querschnittsgestalt aufweist. Eine Verdickung der Kontur zur Anpassung an die sich trichterförmig erweiternde Gestalt des Knochens in dem trochanteren Femurbereich ist nicht vorgesehen. Zwar können sich die Querschnittsräume zwischen den Rippen mit nachwachsendem Knochengewebe füllen; jedoch stellen die ventralen und dorsalen Rippen im Vergleich mit solchen rippenfreien Prothesen, die in der Projektion auf die Anterior-Posterior-Ebene eine ebenso große Schaftfläche besitzen, keine zusätzliche Kraftübertragungsfläche zur Verfügung. Im Gegenteil kann eine bei der bekannten Prothese nach medial weisende Rippe eine nachteilige Aufteilung des medial vom proximalen Schaftabschnitt gelegenen, hochbelasteten Knochengewebes und damit eine Schwächung desselben bewirken.

Ferner ist eine Femurprothese bekannt (US-A- 27 19 522), bei der der proximale Endabschnitt des Prothesenschafts konisch verdickt ist, um eine größere Kraftübertragungsfläche zur Verfügung zu stellen, die der trichterförmigen Erweiterung im trochanteren Femurbereich Rechnung trägt. Der verdickte Schaftteil trägt auf seiner medialen und lateralen Seite eine Vielzahl von flachen Nuten und Rippen, die wahrscheinlich der Drehsicherung des Prothesenschafts im Knochen dienen sollen aber die Übertragung von Kräften vom Prothesenschaft auf den Knochen in der Medial-Lateral-Ebene nicht erleichtern.

Bekannt sind auch Prothesenschäfte (US-A- 3 067 740 und 3 740 769), die sich von distal nach proximal gleichmäßig verdicken und daher im trochanteren Femurbereich eine Querschnittsgröße aufweisen, die bei weitem nicht an die Querschnittsgröße des spongiösen Knochens herankommt. Die Querschnittsgestalt des Schafts ist kreuzförmig, so daß dorsal und ventral Rippen entstehen, die von einem vergleichsweise schlanken Schaftkern vorspringen und der medialen Seite eine Breitfläche zukehren, die zur Krafteinleitung in das Knochengewebe zur Verfügung steht. Jedoch läßt dieser Stand der Technik keine Mittel erkennen, die die Kraftübertragung vom proximalen Endabschnitt des Prothesenschafts nach medial auf den Knochen im Sinne geringerer spezifischer Belastungen und höherer Schaftelastizität verbessern könnten.

Nach dem DE-U- 81 24 912 sind die ventralen und dorsalen Flächen des Prothesenschafts mit flachen Noppen besetzt, die bei zementfreier Verankerung eine Verkeilung des Prothesenschafts im Knochen und seine dauerhafte Festlegung bezwecken. Da jedoch ein Teil der von der Gewichtsbelastung durch den menschlichen Körper herrührenden Kräfte im Trochanterbereich nach der medialen Seite hin durch Druck und nach der lateralen Seite hin durch Zug übertragen werden muß, kann nicht erwartet werden, daß diese Noppen die Kraftübertragungsverhältnisse im Trochanterbereich verbessern, da ihre medial gerichteten Flächenanteile im Vergleich mit der Größe der medialen Schaftstirn, die für die Übertragung der Druckkräfte hauptsächlich verantwortlich ist, zu gering sind.

Nach der CH-A- 622 423 ist der proximale Endabschnitt des Prothesenschafts mit fischgrätartig verlaufenden Erhöhungen versehen, die beim Eintreiben des Schafts in die operativ vorbereitete Knochenhöhlung die Verteilung des verdrängten, spongiösen Knochengewebes verbessern und damit die Verankerung des Schafts durch rasches Einwachsen beschleunigen sollen. Eine Rolle bei der Übertragung der im proximalen Endabschnitt nach medial gerichteten Kräfte ist ihnen offenbar nicht zugedacht, wozu sie aufgrund ihrer Form auch kaum geeignet sind.

Der Erfindung liegt die Aufgabe zugrunde, die Kraftübertragungsverhältnisse im proximalen Endabschnitt des Prothesenschafts zu verbessern.

Die erfindungsgemäße Lösung besteht darin, daß bei einer Hüftgelenkprothese der eingangs genannten Art auf der dorsalen und ventralen Seite des Schaftkerns je eine Mehrzahl von Längsrippen vorspringt, die dem proximalen Abschnitt des Schafts eine zur weitgehenden Füllung des spongiösen Teils des trochanteren Femurbereichs gegenüber dem distalen Abschnitt stark verdickte Kontur verleihen, und daß die in den Längsrippen vorgesehenen Öffnungen zum Rippenrücken hin im wesentlichen geschlossen sind.

Über ihre der medialen Seite zugekehrte Breitfläche können die Rippen zusätzlich zur Schaftoberfläche nach medial gerichtete Kräfte übertragen und verringern dadurch die spezifische Belastung des Knochengewebes in dieser Richtung.

Die Öffnungen der Rippen verringern die Wirksamkeit der Kraftübertragung nicht, weil die einzelnen Öffnungen in Folge ihrer Vielzahl klein sind und das in sie hineinwachsende Gewebe daher festhalten und es an der Kraftübertragung beteiligen. Nicht nur die am weitesten medial gelegene Rippe auf der dorsalen bzw. ventralen Seite des Schafts beteiligt sich an der Kraftübertragung nach medial; vielmehr gilt dies auch für die folgende Rippe bzw. folgenden Rippen, weil diese mit ihren nach medial gerichteten Breitflächen auf das zwischen die Rippen einwachsende Knochengewebe kraftübertragend einwirken. Von diesem aus können die Kräfte unproblematisch an die den Prothesenschaft umgebenden, härteren Querschnittsteile des Knochens weitergegeben werden, weil die Rippen keine Aufteilung des Knochengewebes bewirken; denn das zwischen den Rippen befindliche Knochengewebe steht über die Vielzahl der Rippendurchbrechungen mit dem übrigen Knochengewebe abstützend in Verbindung. Die Öffnungen bezwecken zusätzlich zu ihrer Aufgabe, die beiderseits einer Rippe liegenden Knochenbereiche abstützend zu verbinden, die Steifheit der Rippen gegenüber Biegebeanspruchung des Schaftes zu verringern, um dadurch seine Elastizität zu erhöhen. Ihre Anordnung kann nach bekannten statischen Grundsätzen so gewählt sein, daß diesem Ziel am besten entsprochen wird. Die Verringerung der Rippensteifheit gelingt weitgehend,wenn die Öffnungen zum Rippenrücken hin (das ist die Fläche der Rippe, die vom Schaft wegweist) zumindest teilweise geöffnet sind. Damit die Rippen jedoch nicht nur mit ihren Breitseiten und mit ihren Rücken Druckkräfte, auf das Gewebe übertragen können sondern auch im Bereich ihrer Öffnungen Zugkräfte, dürfen nach der Erfindung die Öffnungen zum Rippenrücken hin nicht vollständig geöffnet sein. Sie sind im Sinne der Erfindung im wesentlichen geschlossen, wenn ein wesentlicher Teil ihrer näher dem Rippenrücken gelegenen Begrenzungsfläche geschlossen ist. Beispielsweise ist es möglich, alle oder einzelne Durchbrechungen durch einen dünnen Schlitz zum Rippenrücken hin zu öffnen und dadurch die Rippensteifigkeit zu verringern, ohne daß dadurch ihr geschlossener Charakter verlorengeht.

Zweckmäßigerweise ist auch auf der lateralen Schaftkernseite im proximalen Endabschnitt des Prothesenschafts mindestens eine Rippe mit zum Rippenrücken hin im wesentlichen geschlossenen Öffnungen vorgesehen, durch die bewirkt werden soll, daß der laterale Knochenbereich beim größeren Trochanter durch Übertragung von Zugkräften von der Prothese auf diesen Knochenbereich an der Kraftübertragung beteiligt wird. Dadurch wird einerseits der mediale Knochenbereich entlastet und andererseits der laterale Knochenbereich vor Rückbildungserscheinungen, die auf zu starke Kraftentlastung zurückzuführen sind, weitgehend geschützt.

Die mediale Seite des proximalen Endabschnitts des Prothesenschafts kann nach der Erfindung im wesentlichen rippenfrei sein, um die Einheitlichkeit des hoch belasteten, medial gelegenen Knochenbereichs nicht zu stören. Kleinere Erhöhungen oder Vertiefungen werden von dieser Überlegung nicht betroffen.

Die Mittelachsen von einander an der ventralen oder dorsalen Seite des Schafts benachbarten Rippen schließen zweckmäßigerweise im Querschnitt einen Winkel von nicht mehr als 30° miteinander ein, wie es auch in der Teilanmeldung 85 113 973.3 (EP-A- 0 181 586) beansprucht wird und sind weiter vorzugsweise sogar etwa parallel zueinander gerichtet, um dadurch einen um so innigeren Verbund des in den Zwischenraum einwachsenden Knochengewebes mit den Rippen zu erzielen. Dies schließt nicht aus, daß die Rippen am Rand im Querschnitt abgerundet sind, um einen harmonischen Kräfteverlauf im Knochengewebe zu erreichen. Dem gleichen Zweck dient es, wenn nach der Erfindung einander benachbarte Rippen an der dorsalen bzw. ventralen Seite des Schafts einen Zwischenraum einschließen, dessen auf den trochanteren Femurbereich bezogene mittlere Querschnittstiefe mindestens etwa seiner Weite gleicht. Dadurch wird nämlich die Verzahnung des Schafts mit dem Knochengewebe verbessert.

Es kann sein, zweckmäßig sein auf der dorsalen und der ventralen Seite des proximalen Endabschnitts des Schafts jeweils zwei Rippen vorzusehen. Insbesondere im endnahen, dickeren Schaftbereich können beispielsweise auch jeweils drei Rippen vorgesehen sein.

Die lichten Querabmessungen der Öffnungen sind vorteilhafterweise nicht größer als etwa 5 mm, vorzugsweise nicht mehr als 3 mm, um einen innigen Verbund mit dem Knochengewebe und insbesondere auch eine Übertragung von Zugkräften von dem Schaft auf das Knochengewebe zu ermöglichen. Sie können beispielsweise als Bohrungen mit rundem lichten Querschnitt ausgebildet sein.

Das Merkmal, daß die Rippen in Längsrichtung des Schaftes verlaufen, ist zum einen dadurch begründet, daß sie der medialen Seite Breitflächen zukehren sollen. Zum anderen werden dadurch Operation und Reoperation erleichtert.

Ein wesentliches Merkmal der Erfindung besteht darin, daß die Querschnittshöhe der Rippen so groß bemessen ist, daß eine Anpassung an unterschiedliche Femurabmessungen durch Abtragung vom Rippenrücken erzielbar ist. Man erhält daher Endoprothesen mit in die Markhöhle von Knochen einzusetzenden Halteschäften von unterschiedlicher Dicke nach der Erfindung dadurch daß ausgehend von einer Standardform eines mit vorstehenden Rippen versehenen Halteschafts stets gleicher Gestalt lediglich die Rippenrücken zur Anpassung an eine individuelle Femurform bearbeitet sind. Auf diese Weise läßt sich eine Vielzahl von Standardgrößen unter Verwendung nur weniger Schaftrohlinge bereitstellen. Ein Verfahren zum Herstellen von Endoprothsen aus Rohlingen gleicher Gestalt, bei dem die Rohlinge mit von einem Schaftkern vorstehenden Rippen hergestellt und die Rippenrücken bearbeitet werden, wird in der Teilanmeldung 85 113 974.1 (EP-A- 0 182 176) beansprucht.

Die Vorteile der Erfindung bestehen vornehmlich darin, daß die Auflösung des Schaftquerschnitts in eine Mehrzahl von Rippen zum einen eine weitgehend willkürliche Bemessung des Widerstandsmoments und damit eine Anpassung der Elastizität an diejenige des Knochens gestattet und daß zum anderen die für den Verbund mit dem vorhandenen bzw. einwachsenden Knochengewebe zur Verfügung stehende Oberfläche in der Hauptbelastungsrichtung vergrößert wird, so daß ein fester Prothesensitz erzielt werden kann. Das in die Zwischenräume der Rippen einwachsende, spongiöse Knochengewebe nimmt dank inniger Verankerung an der Kraftübertragung und der Ernährung des Knochens teil.

Der Begriff Rippen beschränkt die Querschnittsgestalt der so bezeichneten Prothesenteile nicht. Im allgemeinen setzt er jedoch voraus, daß die Rippen zumindest auf einem wesentlichen Teil ihrer Länge eine Querschnittsgestalt mit größerer Abmessung in ihrer von dem Schaftkern nach außen strebenden Hauptrichtung als quer dazu aufweisen.

Das Merkmal, daß die Rippen in Längsrichtung verlaufen, besagt nicht, daß die Rippenrücken in dieser Richtung ununterbrochen ausgebildet sein müßten, jedoch müssen die in den Längsrippen vorgesehenen Öffnungen zum Rippenrücken hin im wesentlichen geschlossen sein. Es können von außen nach innen geführte Vertiefungen, Schlitze oder Unterbrechungen zur Beeinflussung der statischen Festigkeit der Rippen vorteilhaft sein. Zur Beeinflussung ihrer Längsnachgiebigkeit kann auch ein wabenartiger Aufbau der Rippen vorgesehen sein. Der Rippenrücken kann mit Erhöhungen und Vertiefungen als Zahnung ausgebildet sein, die beim Eintreiben des Prothesenschafts sich selbst den Weg freischneidet.

Im Falle einer Reoperation kann es erforderlich sein, die zwischen den Rippen befindliche Knochensubstanz zu entfernen oder mindestens zu lösen. Da die Halsauflage dabei hinderlich sein kann, ist die Prothese halsauflagenlos oder mit nach einem weiteren Merkmal der Erfindung mit einer entfernbaren Halsauflage versehen.

Die Erfindung wird im folgenden näher unter Bezugnahme auf die Zeichnung erläutert, die vorteilhafte Ausführungsbeispiele veranschaulicht. Darin zeigen:

Fig. 1 einen Längsschnitt in der lateral-medial-Ebene eines Oberschenkelknochens mit eingesetzter Hüftgelenkprothese,

Fig. 2 einen Schnitt derselben Anordnung in der anterior-posterior-Ebene,

Fig. 3 eine der Fig. 2 entsprechende Schnittansicht mit einer anderen Prothesenform,

Fig. 4 einen Horizontalschnitt durch Knochen- und Prothesenschaft nahe dem proximalen Schaftende,

Fig. 5 eine der Fig. 1 entsprechende Teilansicht einer Prothese mit lösbarer Halsauflage,

Fig. 6 und 7 eine Schnittansicht und eine Draufsicht auf die Halsauflage,

Fig. 8 einen der Fig. 4 ähnlichen Schnitt durch eine andere Ausführungsform und

Fig. 9 und 10 Teildarstellungen von Rippen mit unterschiedlichen Formen von Öffnungen.

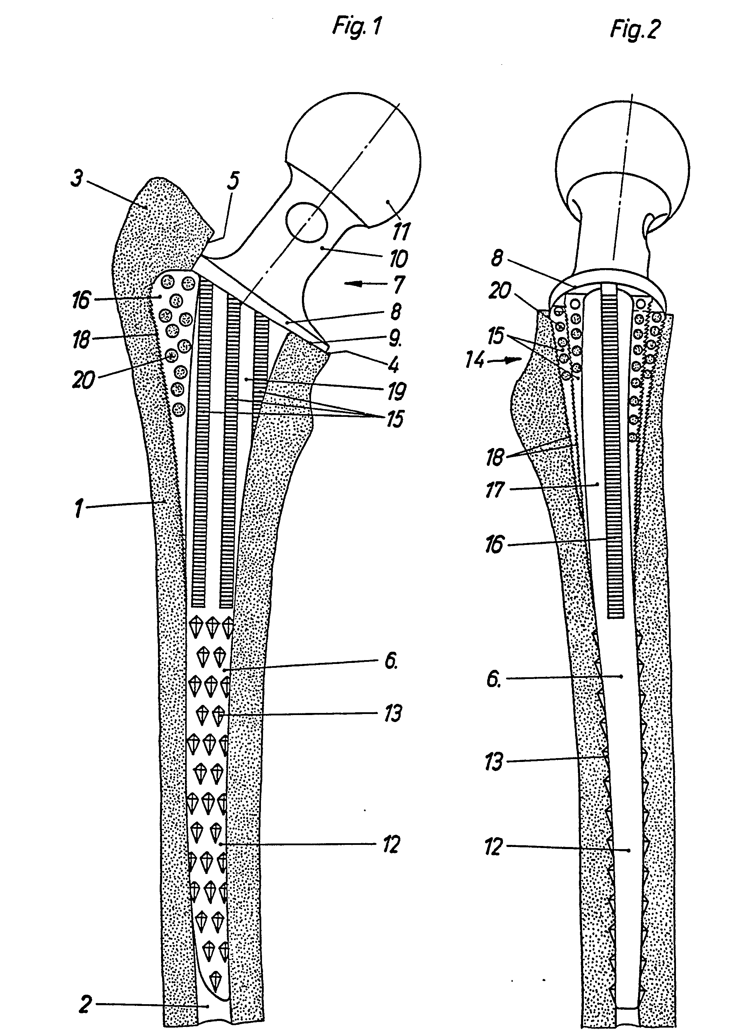

Die Schnittansichtsflächen des Oberschenkelknochens 1 sind in der Zeichnung punktiert angelegt. Man erkennt die Markhöhle 2, in Fig. 1 lateral den großen Trochanter 3 sowie die Resektionsflächen 4, 5, längs welchen der Oberschenkelhals mit dem Gelenkkopf entfernt ist.

In der ausgeräumten Markhöhle 2 befindet sich der zementlos eingesetzte Schaft 6 der Prothese 7. Der Schaft 6 wird proximal durch die Halsauflage 8 abgeschlossen, deren Unterfläche 9 auf der Resektionsfläche 4 aufliegt. Es schließt sich der Hals 10 mit dem Gelenkkopf 11 an. In den Ausführungsbeispielen der Fig. 1 bis 3 ist vorausgesetzt, daß alle diese Prothesenteile einstückig aus demselllben Material, beispielsweise geschmiedetem Titan, bestehen.

Der Prothesenschaft 6 ist in seinem unteren Bereich 12 mit geschlossener Oberfläche ausgeführt, die zwecks besseren Verbundes mit dem Knochen mit Noppen 13 irgendeiner geeigneten Form versehen sein kann. In diesem Bereich ist die Markhöhle des Knochens vergleichsweise eng und individuell weniger unterschiedlich, so daß keine große Zahl variierender Prothesenformen erforderlich ist.

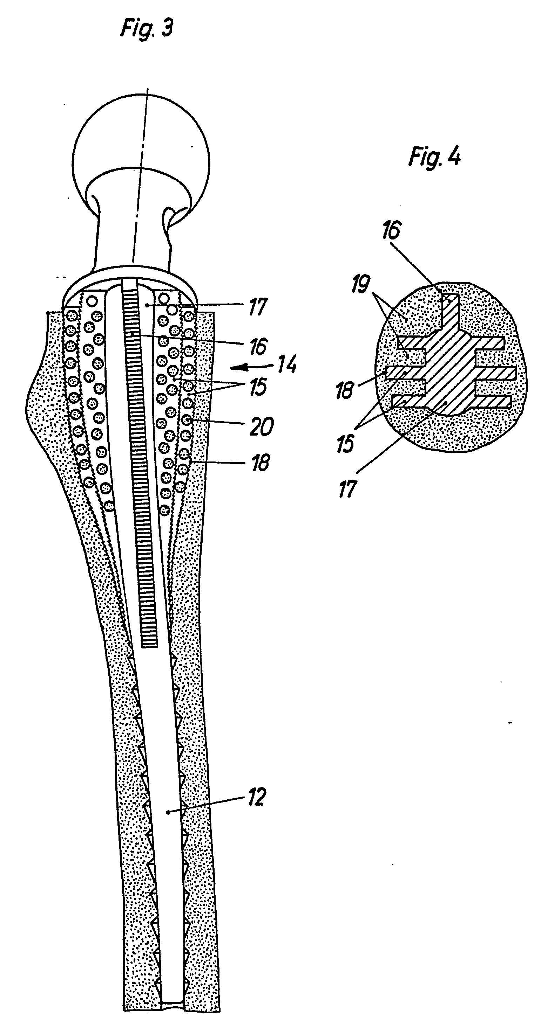

Am proximalen Ende erweitert sich die Markhöhle 2 in dem Bereich, der allgemein mit der Bezugsziffer 14 angedeutet ist, im allgemeinen trompetenartig wie dies in den Fig. 1 und 2 vorausgesetzt wurde. Es kommen jedoch auch andere Erweiterungsformen vor, wie dies beispielsweise in Fig. 3 gezeigt ist. Um der Notwendigkeit zu entgehen, den Schaft 6 in diesem erweiterten Bereich massiv und in einer großen Zahl von Formvariationen entsprechend der individuellen Innengestalt der Corticalis zu gestalten, weist der Prothesenschaft eine Mehrzahl von Rippen 15, 16 auf, die von dem vergleichsweise dünnen Schaftkern 17 im Querschnitt weit nach außen vordringen und sich teilweise der Corticalis nähern. Zumindest füllt der von den Rippen bestimmte Prothesenquerschnitt den spongiösen Teil des trochanteren Femurbereichs großenteils aus. Die Tiefe der Rippen (Hauptabmessung von ihrer Rückenfläche 18 bis zum Schaftkern 17) ist am proximalen Ende am größten, während sie distal allmählich bis auf Null verschwindet.

Im Querschnitt (Fig. 4) sind in den gegebenen Beispielen sechs Rippen 15 auf gegenüberliegenden Seiten parallel zueinander angeordnet, während eine Rippe 16 quer dazu verläuft. Diese Anordnung gestattet die Herstellung durch Schmieden. Wenn ein anderes Herstellungsverfahren gewählt wird, können die Rippen auch in anderer Anordnung verlaufen. Wichtig ist jedoch stets, daß zumindest die am weitesten medial gelegenen Rippen ausgeprägte, nach medial gerichtete Kraftübertragungsflächen bilden. In einem wesentlichen Teil ihrer Länge ist ihre Tiefenabmessung größer als ihre Breite oder wenigstens etwa dieser gleich. Zwischen den Rippen 15, 16 befinden sich Zwischenräume 19, in welchen das vorhandene Knochengewebe verbleiben oder neues sich bilden kann. Die Rippen sind mit einer Vielzahl von Querbohrungen 20 versehen, die die Widerstandskraft der Rippen gegenüber Längskräften herabsetzen und diese dadurch nachgiebiger machen. Dieser Effekt wird in denjenigen Bereichen, in welchen die Rippen besonders tief sind, durch versetzte Anordnung der Öffnungen verstärkt. Außerdem kann Knochengewebe in die Öffnungen hineinwachsen und dadurch die Verankerung des Prothesenschafts im Knochen verbessern und auch die Übertragung von Zugkräften gestatten.

Die Rückenflächen 18 der Rippen sind gezahnt, was nicht nur dem formschlüssigen Verbund mit dem Knochengewebe dienen soll, sondern beim Einsetzen in den Röhrenknochen einen der Arbeitsweise einer Räumnadel gleichenden Effekt haben kann, durch den bei entsprechend knapper Vorbearbeitung des Knochenhohlraums gewährleistet wird, daß die Rückenflächen der Rippen ohne Zwischenraum am Knochengewebe anliegen.

Die Rippen können entsprechend der Richtung der Einsetzbewegung des Prothesenschafts in den Knochen gekrümmt sein, damit möglichst wenig Knochensubstanz vor dem Einsetzen bzw. während desselben weggeräumt werden muß.

Durch die ausgedehnten, mit dem Knochengewebe zusammenwirkenden Seitenflanken der Rippen 15 wird die Kraftübertragung nach medial begünstigt. Die laterale Rippe 16, die in bekannter Anordnung in dem Trochanter eingreift, beteiligt auch diesen an der Kraftübertragung. Zusätzlich können weitere Mittel zur Übertragung von Kräften vom Prothesenschaft oder von der Halsauflage 8 auf die lateralen Bereiche des Knochens vorgesehen sein.

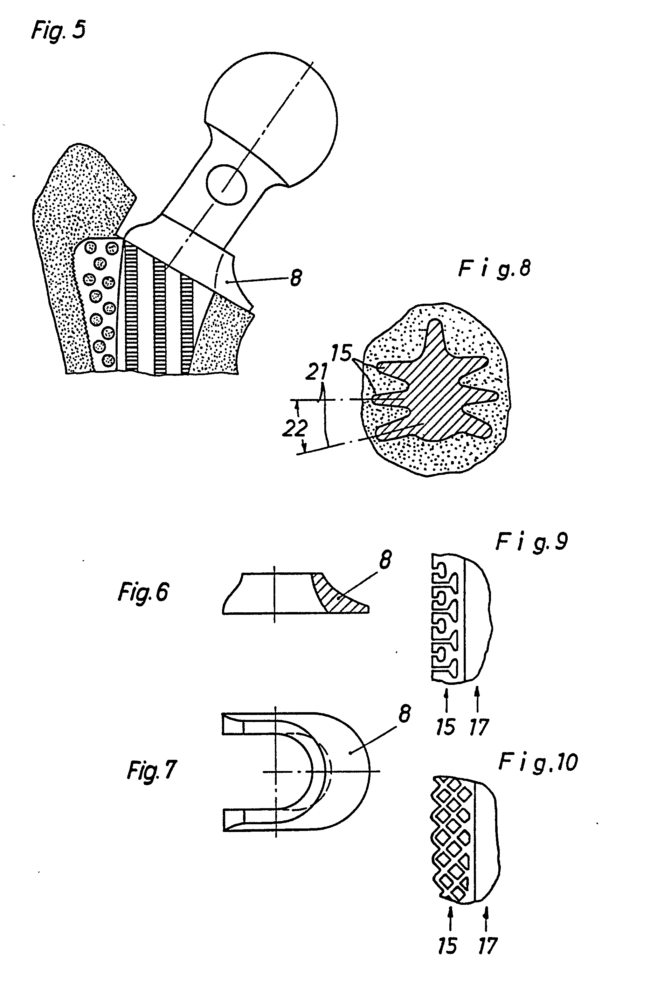

Die Längsnachgiebigkeit der Rippen kann durch seitliche Einschnitte vergrößert werden. Beispielsweise ist in Fig. 9 vorgesehen, daß die Löcher 20 sämtlich oder teilweise durch einen Schlitz mit der Rippenoberfläche 18 verbunden sind. Dadurch wird das Widerstandsmoment des oberen Prothesenteils im wesentlichen auf dasjenige des Schaftkerns 17 reduziert. An den Außenflächen 18 der Rippen finden dann Kompressions- und Dehnbewegungen statt, die für ein gegebenes Biegemoment denjenigen der natürlichen Knochensubstanz gleichen können.

Gemäß Fig. 10 ist vorgesehen, daß die Öffnungen wabenartig ausgebildet und angeordnet sind, derart daß sie von dünnen, schräg zur Schaftlängesrichtung verlaufenden Stegen getrennt sind, die so bemessen werden können, daß sich die gewünschte Schaftelastizität ergibt.

Während die Halsauflage 8 in den Fig. 1 bis 3 einstückig mit dem Prothesenschaft vorausgesetzt wurde, besteht sie im Fall der Fig. 5 bis 7 aus einem besonderen, hufeisenförmigen Teil, das nach dem Einsetzen der Prothese mit dieser auf nicht gezeigte Weise fest aber lösbar verbunden werden kann. Dies ermöglicht im Falle einer Reoperation leichteren Zugang zu den Rippenzwischenräumen 19 zum Lösen des dortigen Verbundes mit dem Knochengewebe.

Man erkennt, daß Prothesen mit unterschiedlicher Rippenform aus gleichen Rohlingen durch entsprechend unterschiedliches Abfräsen der Rippenrücken erzeugt werden können. Zum Beispiel können auch die Ausführungsbeispiele der Fig. 2 und 3 aus gleichen Rohlingen erzeugt sein. Es stört nicht, wenn durch den Materialabtrag von den Prothesenrücken einige der Öffnungen vollständig geöffnet werden, sofern nur andere Öffnungen erhalten bleiben, die zum Rippenrücken im wesentlichen geschlossen sind und dadurch Zugkräfte zwischen der Prothese und dem umgebenden Knochenmaterial durch Einwirkung auf das in sie eingewachsene Knochenmaterial übertragen können.

In Fig. 8 ist ein Prothesenquerschnitt gezeigt, in welchem die Mittelachsen 21 benachbarter Rippen 15 einen engen Winkel 22 miteinander einschließen, der kleiner als 30° ist. Der zwischen ihnen gebildete Raum 19 stellt - ebenso wie im Beispiel der Fig. 4 - eine Kammer dar, in der Knochengewebe sich verankern kann.

Als Vorteil der erfindungsgemäßen Prothese ist es zu werten, daß die Rippenrücken 18, die sich nach der Operation im unmittelbaren Kontakt mit Knochengewebe befinden sollen, eine vorläufig ausreichende Kraftübertragung bei mäßiger Belastung ermöglichen. Die volle Belastbarkeit stellt sich mit dem Einwachsen des Knochens in die Rippenzwischenräume 19 ein.

Die mittlere Weite der Zwischenräume sollte im allgemeinen nicht kleiner als 2 mm sein. Als zweckmäßig hat sich eine mittlere Weite von etwa 3 mm erwiesen.

**Patentansprüche**

1. Femorale Hüftgelenkprothese (7) mit einem zur zementfreien Verankerung im Femur (1) geeigneten Schaft (6), der eine Mehrzahl von von einem schlanken Schaftkern (17) vorspringenden Längsrippen (15, 16) aufweist, von denen die dorsal und ventral vorgesehenen Rippen (15) der medialen Seite eine Breitfläche zukehren und eine Vielzahl von Öffnungen (20) enthalten, dadurch gekennzeichnet, daß auf der dorsalen und ventralen Seite des Schaftkerns (17) je eine Mehrzahl von Längsrippen (15) vorspringt, die dem proximalen Abschnitt des Schafts (6) eine zur weitgehenden Füllung des spongiösen Teils des trochanteren Femurbereichs (14) gegenüber dem distalen Abschnitt (12) stark verdickte Kontur verleihen, und daß die in den Längsrippen vorgesehenen Öffnungen (20) zum Rippenrücken (18) hin im wesentlichen geschlossen sind.

2. Prothese nach Anspruch 1, dadurch gekennzeichnet, daß mindestens eine Rippe (16) mit zum Rippenrücken hin im wesentlichen geschlossenen Öffnungen (20) auf der lateralen Schaftkernseite vorgesehen ist.

3. Prothese nach Anspruch 1 oder 2, dadurch gekennzeichnet, daß die mediale Seite rippenfrei ist.

4. Prothese nach einem der Ansprüche 1 bis 3, dadurch gekennzeichnet, daß die Mittelachsen von einander an der ventralen oder dorsalen Seite des Schafts benachbarten Rippen (15) Querschnitt einen Winkel (22) von nicht mehr als 30° miteinander einschließen.

5. Prothese nach Anspruch 4, dadurch gekennzeichnet, daß die Rippen (15) im Querschnitt zueinander etwa parallel gerichtet sind.

6. Prothese nach einem der Ansprüche 1 bis 5, dadurch gekennzeichnet, daß einander benachbarte Rippen (15) an der dorsalen bzw. ventralen Seite des Schafts einen Zwischenraum (19) einschließen, dessen Tiefe quer zur Schaftlängsrichtung im Trochanterbereich mindestens etwa seiner Weite gleich ist.

7. Prothese nach einem der Ansprüche 1 bis 6 dadurch gekennzeichnet, daß auf der dorsalen und der ventralen Seite jeweils drei Rippen vorgesehen sind.

8. Prothese nach einem der Ansprüche 1 bis 7, dadurch gekennzeichnet, daß die Öffnungen (20) eine lichte mittlere Abmessung von nicht mehr als 5 mm aufweisen.

9. Prothese nach Anspruch 8, dadurch gekennzeichnet, daß die Abmessung nicht mehr als 3 mm beträgt.

10. Prothese nach einem der Ansprüche 1 bis 9, dadurch gekennzeichnet, daß die Öffnungen (20) als Bohrungen mit rundem lichten Querschnitt ausgebildet sind.

11. Prothese nach einem der Ansprüche 1 bis 10, dadurch gekennzeichnet, daß die Höhe der Rippen quer zur Schaftlängsrichtung so groß bemessen ist, daß eine Anpassung an unterschiedliche Femurabmessungen durch Abtragung von den Rippen erzielbar ist.

12. Prothese nach einem der Ansprüche 1 bis 11, dadurch gekennzeichnet, daß eine entfernbare Halsauflage (8) vorgesehen ist.

13. Prothese nach einem der Ansprüche 1 bis 12, dadurch gekennzeichnet, daß ausgehend von einer Standardform stets gleicher Gestalt lediglich die Rippenrücken (18) zur Anpassung an eine individuelle Femurform bearbeitet sind.

**Claims**

1. A femoral hip-joint prosthesis (7) having a shaft (6) suitable for uncemented anchoring in the femur (1), which shaft has a plurality of longitudinal ribs (15, 16) projecting from a slender shaft core (17), of which the dorsally and ventrally provided ribs (15) offer a broad surface to the medial side and comprise a plurality of openings (20), characterised in that on the dorsal and ventral side of the shaft core (17) there projects respectively a plurality of longitudinal ribs (15), which impart to the proximal portion of the shaft (6) a considerably thickened contour in relation to the distal portion (12) so as to substantially fill the spongy part of the trochanter femoral region (14), and in that the openings (20) provided in the longitudinal ribs (20) are substantially closed towards the rib back (18).

2. A prosthesis according to Claim 1, characterised in that at least one rib (16) is provided on the lateral shaft core side with openings (20) which are substantially closed towards the rib back.

3. A prosthesis according to Claim 1 or 2, characterised in that the medial side is free of ribs.

4. A prosthesis according to any of Claims 1 to 3, characterised in that the centre axes of mutually adjacent ribs (15) on the ventral or dorsal side of the shaft together include in cross-section an angle (22) of not more than 30°.

5. A prosthesis according to Claim 4, characterised in that in cross-section the ribs (15) are directed substantially parallel to one another.

6. A prosthesis according to any of Claims 1 to 5, characterised in that mutually adjacent ribs (15) on the ventral and/or dorsal side of the shaft together form an intermediate space (19), the depth of which transversely to the longitudinal direction of the shaft in the trochanter region is at least substantially equal to its width.

7. A prosthesis according to any of Claims 1 to 6, characterised in that three ribs are provided on the dorsal and ventral side respectively.

8. A prosthesis according to any of Claims 1 to 7, characterised in that the openings (20) have a mean inside dimension of not more than 5 mm.

9. A prosthesis according to Claim 8, characterised in that the dimension is not more than 3 mm.

10. A prosthesis according to any of Claims 1 to 9, characterised in that the openings (20) are in the form of bores with circular inside cross-section.

11. A prosthesis according to any of Claims 1 to 10, characterised in that the height of the ribs transversely to the longitudinal direction of the shaft is of such a size that an adjustment to different femoral dimensions can be achieved by removing ribs.

12. A prosthesis according to any of Claims 1 to 11, characterised in that a removable neck support (8) is provided.

13. A prosthesis according to any of Claims 1 to 12, characterised in that starting from a standard shape of always uniform design only the rib backs (18) are machined for adjustment to an individual femur form.

**Revendications**

1. Prothèse fémorale de la hanche (7), comportant une tige (6) se prêtant à un ancrage sans ciment dans le fémur (1) et présentant plusieurs nervures longitudinales (15, 16) qui font saillie sur une âme de tige de forme elancée (17) et parmi lesquelles les nervures (15) prévues sur la face dorsale et ventrale dirigent une de leurs grandes surfaces du côté interne et contiennent une multiplicité d'ouvertures (20), caractérisée en ce que font saillie, sur chacun des côtés dorsal et ventral de l'âme de tige (17), plusieurs nervures longitudinales (15) qui donnent au segment proximal de la tige (6) un contour fortement epaissi en comparaison du segment distal (12) afin de remplir dans une large mesure la partie spongieuse de la région trochantérienne (14) du fémur, et en ce que les ouvertures (20) prévues dans les nervures longitudinales sont pratiquement fermées vers le dos (18) de ces nervures.

2. Prothèse selon la revendication 1, caractérisée en ce qu'il est prévu, sur le côté latéral de l'âme de tige, au moins une nervure (16) dont les ouvertures sont pratiquement fermées vers le dos de la nervure.

3. Prothèse selon la revendication 1 ou 2, caractérisée en ce que le côté interne est exempt de nervures.

4. Prothèse selon l'une quelconque des revendications 1 à 3, caractérisée en ce que les axes de nervures (15) voisines l'une de l'autre sur le côté ventral ou dorsal de la tige forment entre eux, en coupe transversaie, un angle (22) qui ne dépasse pas 30°.

5. Prothèse selon la revendication 4, caractérisée en ce que les nervures (15) sont dirigées à peu près parallèlement entre elles en coupe transversale.

6. Prothèse selon l'une quelconque des revendications 1 à 5, caractérisée en ce que des nervures (15) voisines l'une de l'autre sur le côté dorsal ou ventral de la tige délimitent un intervalle (19) dont la profondeur perpendiculairement à la direction longitudinale de la tige est au moins à peu près égale à sa largeur dans la région trochanterienne.

7. Prothèse selon l'une quelconque des revendications 1 à 6, caractérisée en ce qu'il est prévu trois nervures sur chacun des côtés dorsal et ventral.

8. Prothèse selon l'une quelconque des revendications 1 à 7, caractérisée en ce que les ouvertures (20) présentent un calibre intérieur moyen qui ne dépasse pas 5 mm.

9. Prothèse selon la revendication 8, caractérisée en ce que le calibre ne s'élève pas à plus de 3 mm.

10. Prothèse selon l'une quelconque des revendications 1 à 9, caractérisée en ce que les ouvertures (20) sont réalisées sous forme de forures de section libre circulaire.

11. Prothèse selon l'une quelconque des revendications 1 à 10, caractérisée en ce que la hauteur des nervures perpendiculairement à la direction longitudinale de la tige est dimensionnée suffisamment grande pour qu'une adaptation à différentes dimensions de fémurs soit réalisable par érosion des nervures.

12. Prothèse selon l'une quelconque des revendications 1 à 11, caractérisée en ce qu'il est prévu un appui amovible de col (8).

13. Prothèse selon l'une quelconque des revendications 1 à 12, caractérisée en ce qu'à partir d'un modèle standard qui a toujours la même forme, il suffis d'usiner les dos (18) des nervures pour son adaptation à une forme individuelle de fémur.

Fig. 1

Fig. 2

Fig. 3

Fig. 4

Fig. 5

*8*

F i g. 8

*15*
*21*
*22*

F i g. 9

Fig. 6

*8*

*15  17*

Fig. 7

*8*

F i g. 10

*15  17*